# EUROPEAN PATENT APPLICATION

(11) **EP 3 808 264 A1**
(43) Date of publication of application: **21.04.2021**
(21) Application number: 20152079.8
(22) Date of filing: 15.01.2020
(51) Int. Cl.: A61B 5/053, A61B 5/00, G03B 17/56

(54) **SKIN DETECTING SYSTEM**

(30) Priority: 18.10.2019 CN 201921756126 U
(71) Applicant: Lifetrons Switzerland Holdings Limited, Tuen Mun (HK)
(72) Inventor: Tseng, Chih-Ming, Tuen Mun (HK)
(74) Representative: Lang, Christian

(57) **Abstract**

A skin detecting system is disclosed. The skin detecting system includes a mobile device and a close-up photography assembly. The mobile device comprises a camera, an image unit, and an interpretative software. The close-up photography assembly, disposed on the camera of the mobile device, includes a light source module and an electrical impedance module. The light source module is provided for providing a light source required for the close-up photography assembly to photograph, and the electrical impedance module provided for detecting oil and water content in a skin of a human being. When the close-up photography assembly cooperates with the camera of the mobile device to photograph the skin of the human bring, the skin of the human bring is irradiated by the light source that is provided by the light source module of the close-up photography assembly, and then an image corresponding to the skin of the human being is formed by the image unit and shown at the mobile device. The interpretative software is provided for adjusting magnification and resolution of the image and is provided for executing an analysis of a skin condition to acquire information corresponding to the skin condition, and the information is shown at the mobile device.

## Description

### FIELD OF THE PRESENT INVENTION

The present invention relates to a skin detecting system, and particularly relates to the skin detecting system for use combined with a mobile device.

### BACKGROUND OF THE PRESENT INVENTION

In response to the trend that the current technological life links with a mobile internet, the development of the mobile device are becoming more widely used for the technological life in addition to the use of communication, such as health management, daily services, remote home supervision, management of household electric appliance. Speaking of skin care, the public is accustomed to relying on beauty equipment, but the beauty equipment is expensive despite high market share so that the popularity of the public is low. Therefore, a skin detecting technology combined with the mobile device has developed in recent years in order to fulfill facility and popularity. In view of the fact that pores of the skin are too small, an ultraviolet light source and lens with variable focal length are most required to acquire acne distribution in the pores. However, the mobile device is certainly lack of the ultraviolet light source and lens with sufficient magnification because its photography equipment is essentially designed for entertainment needs. It is obviously bottleneck of development that a skin image sufficient to show pores and acne is acquired through the photography equipment of the mobile device.

"Background" paragraphs are only intended to illustrate the present invention, and thus the disclosure of the "Background" may contain some known techniques that are not known to a skilled person in the ordinary art. The content disclosed in the "Background" section does not mean that a problem to be solved in one or more embodiments of the present invention have been known or recognized by the skilled person in the ordinary art prior to the application of the present invention.

### SUMMARY OF THE PRESENT INVENTION

According to above drawbacks in the conventional prior art, the present invention provides a skin detecting system which is simple, high resolution, and the images can be identified that are invisible to the vision, especially for the image that reflects the fluorescence response.

It is an objective of the present invention that the skin detecting system includes a mobile device and a close-up photography assembly. The mobile device includes a camera, an image unit, and an interpretative software. The close-up photography assembly, disposed on the camera of the mobile device, includes a light source module and an electrical impedance module. The light source module is provided for providing a light source required for the close-up photography assembly to photograph and the electrical impedance module is provided for detecting oil and water content in a skin. When the close-up photography assembly cooperates with the camera of the mobile device to photograph the skin of the human being, the skin of the human being is irradiated by the light source that is provided by the light source module of the close-up photography assembly, and then an image corresponding to the skin of the human being is formed by the image unit and shown at the mobile device. The interpretative software is provided for adjusting magnification and resolution of the image and is provided for executing an analysis of a skin condition to acquire the information corresponding to the skin condition, and the information is shown at the mobile device.

According to above objective, embodiments of the present invention can photograph the skin by irradiation of an ultraviolet light which has energy exciting epidermal molecules of the skin to produce variation of energy levels, and causing blemishes, texture, and acne on epidermal skin to be expressed by distinct fluorescence response that shows a fluorescent color in the photographed image. The present invention helps the professionals to evaluate the skin condition of participants and facilitates the publics to carry and test by themselves in daily life as the interpretative software modulates and/or analyzes the image.

### BRIEF DESCRIPTION OF THE DRAWINGS

Fig. 1A is a schematic of illustrating a skin detecting system in accordance with the present invention.
Fig. 1B is a block diagram of illustrating a skin detecting system in accordance with the present invention.
Fig. 2 is a side view of illustrating a close-up photography assembly in accordance with the present invention.
Fig. 3 is a schematic of illustrating an interface for users shown in the interpretation software in accordance with the present invention.
Fig. 4A is a schematic of illustrating the skin image photographed through the ultraviolet light in accordance with Example 1 of the present invention.
Fig. 4B is a schematic of illustrating the skin image photographed through the white light in accordance with Example 2 of the present invention.

### DETAILED DESCRIPTION OF THE PREFERRED EMBODIMENT

In order to let one of skilled in the art sufficiently understand the technical contents of a skin detecting system of the present invention, relative embodiments and essential components are provided to illustrate the present invention. However, essential components of skin detecting system in following embodiments do not limit the present invention.

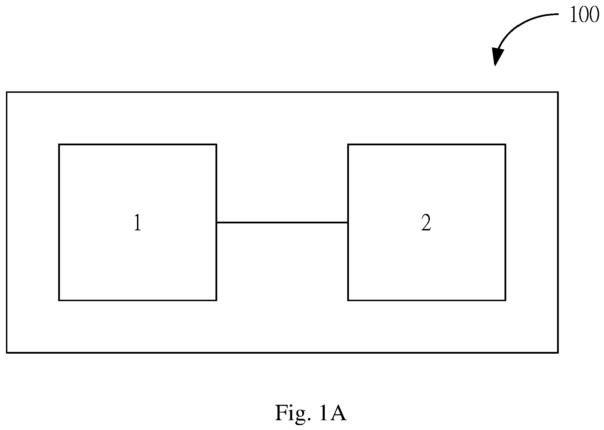

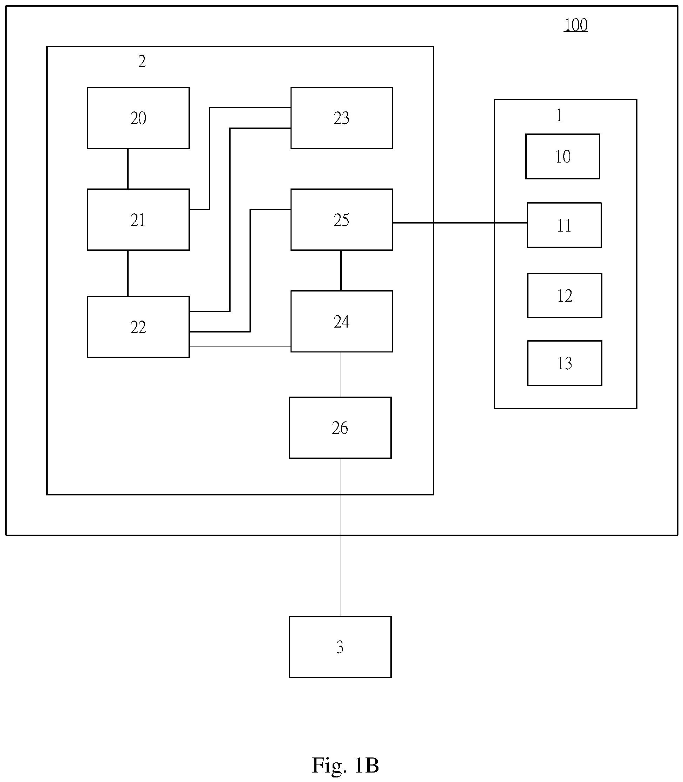

First, please refer to Fig. 1A and Fig. 1B. Fig. 1A is a schematic of illustrating a skin detecting system, and Fig. 1B is a block diagram of illustrating a skin detecting system in accordance with the present invention. In Fig. 1A, a skin detecting system 100 includes a close-up photography assembly 1 and a mobile device 2. In Fig. 1B, the mobile device 2 comprises a camera 20, an image unit 21, and an interpretative software 22. The close-up photography assembly 1 is superimposed on the camera 20 of the mobile device 2 and may connect to the mobile device 2 in electricity/signals through an electric wire 11. Besides, the close-up photography assembly 1 may share electric power with the mobile device 2 through the electric wire 11, or may be individual charged, but not limited in the present invention. Perfectly, the electric wire 11 connects to the mobile device 2 in electricity/signals by means of USB OTG (USB on-the-go) so that the mobile device 2 accomplishes data transmission to the close-up photography assembly 1, and even is capable of charging the close-up photography assembly 1.

In addition, the close-up photography assembly 1 maybe superimposed on the camera 20 of the mobile device 2 by means of clamping. Furthermore, the close-up photography assembly 1 can be set in accordance with the position that the camera 20 is disposed on the mobile device 2. When externally superimposed on the camera 20, the close-up photography assembly 1 may completely overlap the camera 20 so that the camera 20 can obtain images of the skin from the close-up photography assembly 1.

Subsequently, the image unit 21 of the mobile device 2 is used for imaging and provides an ultraviolet light or a white light as a light source for photographing. After the close-up photography assembly 1 cooperates with the camera 20 of the mobile device 2 to photograph the skin of the human being, the image is formed in the image unit 21 and shown on a display unit 23 of the mobile device 2 and is transmitted to the interpretative software 22 installed in the mobile device 2.

The interpretative software 22 is used for modulating magnification and resolution of the image and executing an analysis of a skin condition to acquire the information corresponding to the skin condition, and the information is shown on the display unit 23 of the mobile device 2. The analysis of the skin condition includes data on a pore distribution, a skin oil and water content, a skin elasticity, a skin softness, a skin age index, and texture, blemishes, and acne of the skin. In one embodiment, the information shown at the mobile device 2 corresponds to the data analyzed about the skin condition, and includes evaluation of the skin oil and water content, the skin elasticity, the skin softness, the skin age index, and texture, blemishes, and acne of the skin, and etc.

In one embodiment, the mobile device 2 further comprises a data processing module 24 and a network module 26. The data processing module 24 can transform the data analyzed about the skin condition and the corresponding information into signals transmitted to an outer apparatus 3 by ways of the network module 26, Bluetooth, and/or USB (universal serial bus). The outer apparatus 3 includes a cloud database, a network platform, a detecting instrument, a computer, or other mobile device. By the cloud database storing the data and the information, the professionals can use communication software such as WeChat and email to receive or reply consultation from participants so that remote service is accomplished. Then, the publics can refer to the information and transmit the information to the beauty website platform where they can choose beauty products based on the information and quickly buy beauty products that suit their skin condition during e-shopping.

In one embodiment, the mobile device 2 further comprises a USB connector 25. The data processing module 24 transmits the data analyzed about the skin condition and the corresponding information to the outer apparatus 3 such as the detecting instrument, the computer, or other mobile device through the USB connector 25. Besides, the USB connector 25 may connect to the close-up photography assembly 1 in electricity/ signals through the electric wire 11.

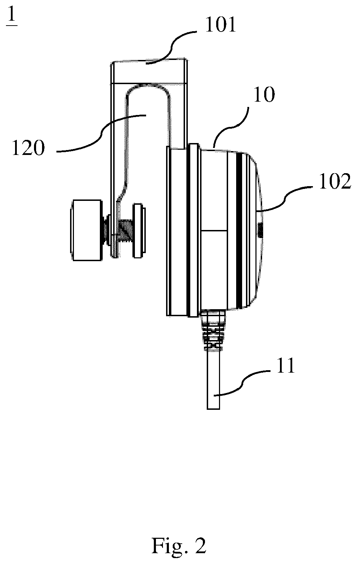

Next, please refer to Fig. 2. Fig. 2 is a side view of illustrating a close-up photography assembly in accordance with the present invention. In Fig. 2, the close-up photography assembly 1 includes a lens 10, the electric wire 11, and a U-shaped fastener 101. The lens 10 respectively connects to the electric wire 11 and the U-shaped fastener 101. A clamping space 120 is configured to locate between the lens 10 and the U-shaped fastener 101. The U-shaped fastener 101 is pulled out in order to dispose the mobile device 2 in the clamping space 120 and to leave the close-up photography assembly 1 clamping at outside of the mobile device 2 and superimposed on the camera 20.

In one embodiment, the close-up photography assembly 1 maybe set a USB receptacle additionally used for connecting to the electric wire 11. In other words, the electric wire 11 may not directly set on the close-up photography assembly 1 but connect to the close-up photography assembly 1 through the USB receptacle. Wherein the electric wire 11 is perfectly a USB cable with one end as a USB plug interposed to the USB receptacle of the close-up photography assembly 1.

The close-up photography assembly 1 is exemplary a macrolens 10 with a perfect magnification ratio of 1:5 ∼ 1:1 and with autofocuses operated by the mobile device 2. The close-up photography assembly 1, which is superimposed on the camera 20 of the mobile device 2 and has the macrolens 10, can focus on the skin of the human being at such close distance that the mobile device 2 can focus on the skin of the human being close through the close-up photography assembly 1 and acquire images showing pores or texture of the skin clearly. In addition, the close-up photography assembly 1 further includes a light source module 12 providing the light source required for photographing, wherein the light source maybe the ultraviolet light or the white light. When the macrolens 10 focuses on the target skin, the light source module 12 is started by the interpretative software 22 to provide the ultraviolet light irradiating the photographed area, and the skin image showing the fluorescent color is photographed through the ultraviolet light. In following, the photographed skin image is executed interpretation by the interpretative software 22. In addition, the close-up photography assembly 1 further includes an electrical impedance module 13 used for contacting with the skin of the human being to execute detection of oil and water content in the skin of the human being. The results of the detection include oil content ratio and water content ratio. The details of the electrical impedance module 13 would be illustrated in following paragraph.

Accordingly, in one embodiment, a user first starts the interpretative software 22 of the mobile device 2 when using the skin detecting system 100 to shoot the skin of the human being. A user interface of the interpretative software 22 may include a shooting mode, a modulating mode, an analysis mode, a storage mode, and a network mode. Wherein the shooting mode comprises a specific image mode, a portrait mode and/or a scene mode. It is noted that the light source module 12 of the close-up photography assembly 1 provides the white light as the light source required for the portrait mode and/or the scene mode when the shooting mode of the interpretation software 22 selects the portrait mode and/or the landscape mode. At this time, the white light can be regarded as has functionality of a flash light or a fill light in the mobile device 2, and then the image unit 21 and the close-up photography assembly 1, the same as an ordinary photography assembly, can obtain images of a man or a scene. Its shooting steps and photography details are not within the scope of the present invention, so no more statements are made.

The light source module 12 receives a white light signal or an ultraviolet light signal that controls light source module 12 to provide the white light or the ultraviolet light from the interpretative software 22 through the electric wire 11. Consequently, when the shooting mode of the interpretation software 22 selects the specific image mode, the close-up photography assembly 1, the camera 20 of the mobile device 2 and the image unit 21 can be started simultaneously. Then the light source module 12 of the close-up photography assembly 1 receive the ultraviolet light signal transmitted from the interpretative software 22 through the electric wire 11 and is started to provide the ultraviolet light as the light source required for the specific image mode. The light source module 12 provides the ultraviolet light to irradiate the skin of the human being and to obtain the skin image showing fluorescent color when the macrolens 10 of the close-up photography assembly 1 focuses on an object to be imaged (such as the skin of the human being). Then, the modulating mode is clicked to modulate magnification and resolution of the skin image acquired from the image unit 21. Then, the analysis mode is clicked to execute evaluation of the skin oil and water content, the skin elasticity, the skin softness, the skin age index, and texture, blemishes, and acne of the skin according to the skin image.

The electrical impedance module 13 of the close-up photography assembly 1 is used for detecting oil and water content and elasticity of the skin of the human being when the close-up photography assembly 1 contacts with the skin of the human being. The detecting results comprise elasticity value, oil content ratio and water content ratio of the skin. The electrical impedance module 13 transmits the detecting results to the USB connector 25 through the electric wire 11 in the forms of skin elasticity signals and oil-water signals that transmitted to the data processing module 24 from the USB connector 25 then, and then transmitted to the interpretation software 22 from the data processing module 24.

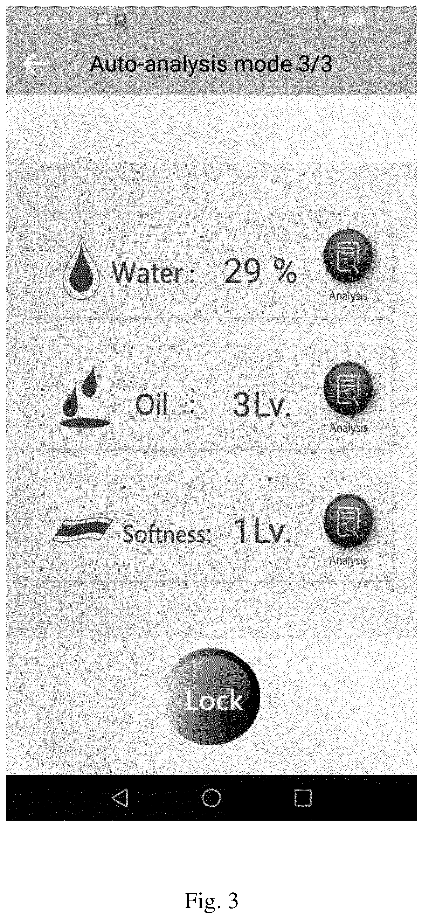

The interpretation software 22 execute analysis of the skin condition according to oil-water signals to acquire the corresponding evaluated information about the skin oil and water content. The evaluated information about the skin oil and water content is expressed at an interface for users on the display unit 23 of the mobile device 2. Finally, the storage mode is clicked to store the data analyzed about the skin condition and the corresponding information as history records so that the interpretation software 22 occupies a lot of memory. In addition, the network mode is clicked in case of required transmission to the cloud database or the network platform through internet network, the interpretation software 22 would transform the data analyzed about the skin condition and the corresponding information into signals. The signals are transmitted to the cloud database or the network platform through the network module 26. Please refer to Fig. 3. Fig. 3 is a schematic of illustrating the interface for users shown in the interpretation software. As shown in Fig. 3, the interpretation software 22 shows the evaluated information about the skin oil content, the skin water content, and the skin softness through the interface for users present on the display unit 23 of the mobile device 2.

### Example 1

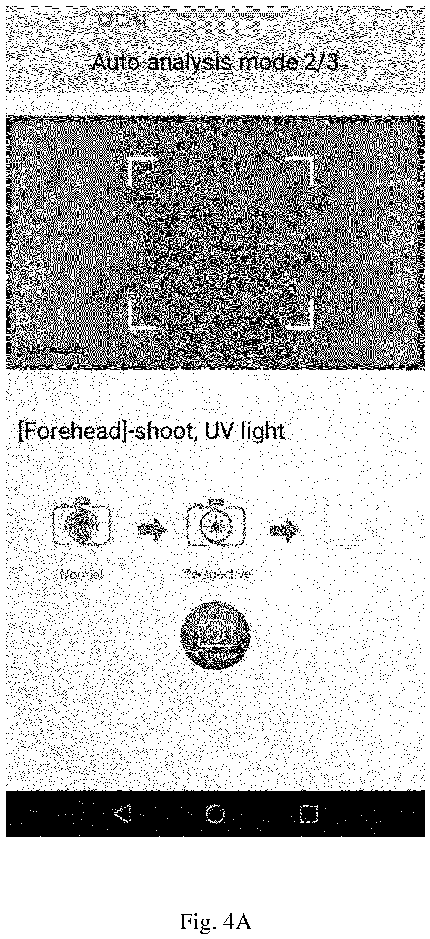

Example 1 refers that users shoot the skin of the human being by using the ultraviolet light. Please refer to Fig. 4A. Fig. 4A is a schematic of illustrating the skin image photographed through the ultraviolet light. In Fig. 4A, after users start the shooting mode of the interpretation software 22 and select the specific image mode, users can see the image where the skin irradiated by the ultraviolet light at the interface for users. Then, users complete shooting by pressing a capture button shown at the interface for users. As a result, the skin image showing the fluorescent color is photographed through the ultraviolet light.

### Example 2

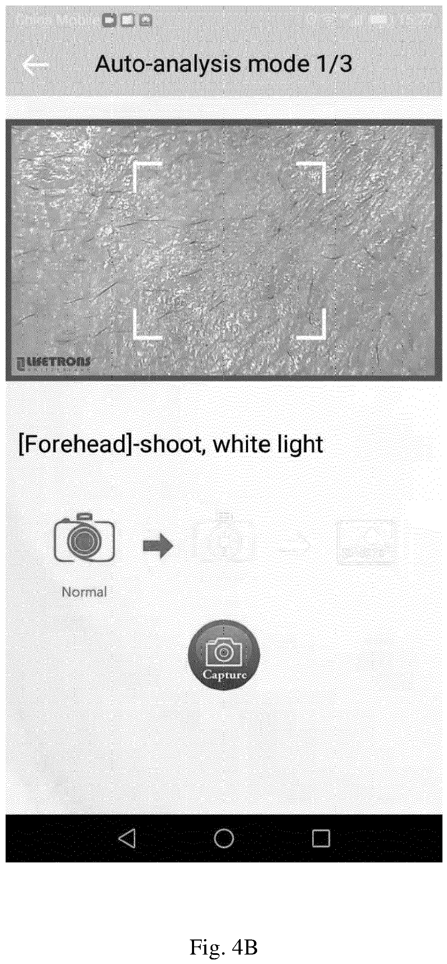

Example 2 refers that users shoot the skin of the human being by using the white light. Please refer to Fig. 4B. Fig. 4B is a schematic of illustrating the skin image photographed through the white light. In Fig. 4B, after users start the shooting mode of the interpretation software 22 and select the portrait mode, users can see the image where the shin irradiated by the white light at the interface for users. Then, users complete shooting by pressing a capture button shown at the interface for users. As a result, the skin image showing the natural color is photographed through the white light.

While the present invention has been described in terms of what is presently considered to be the most practical and preferred embodiments, it is to be understood that the present invention needs not be limited to the disclosed embodiments. On the contrary, it is intended to cover various modifications and similar arrangements comprised within the spirit and scope of the appended claims which are to be accorded with the broadest interpretation so as to encompass all such modifications and similar structures.

## Claims

1. A skin detecting system, **characterized in that** comprising:
a mobile device (2), comprising a camera (20), an image unit (21), and an interpretative software (22); and
a close-up photography assembly (1), disposed on the camera (20) of the mobile device (2), the close-up photography assembly (1) comprises a light source module (12) and an electrical impedance module (13), the light source module (12) is provided for providing a light source required for the close-up photography assembly (1) to photograph and the electrical impedance module (13) is provided for detecting oil and water content in a skin of a human being;
wherein when the close-up photography assembly (1) cooperates with the camera (20) of the mobile device (2) to photograph the skin of the human being, the skin of the human being is irradiated by the light source that is provided by the light source module (12) of the close-up photography assembly (1), and then an image corresponding to the skin of the human being is formed by the image unit (21) and shown at the mobile device (2), and the interpretative software (22) is provided for adjusting magnification and resolution of the image, and is provided for executing an analysis of a skin condition to acquire an information corresponding to the skin condition, and the information is shown at the mobile device (2).

2. The skin detecting system according to claim 1, wherein the light source provided by the light source module (12) is ultraviolet light when the close-up photography assembly (1) cooperates with the camera (20) of the mobile device (2) to photograph the skin of the human being.

3. The skin detecting system according to claim 1, wherein the light source provided by the light source module (12) is white light when the close-up photography assembly (1) cooperates with the camera (20) of the mobile device (2) to photograph the skin of the human being.

4. The skin detecting system according to claim 1, wherein the image unit (21) of the mobile device (2) provides ultraviolet light or white light.

5. The skin detecting system according to claim 1, wherein the close-up photography assembly (1) comprises an electric wire (11) that is provided for electrically connecting to the mobile device (2).

6. The skin detecting system according to claim 1, wherein the close-up photography assembly (1) comprises a macrolens (10).

7. The skin detecting system according to claim 1, wherein the analysis of the skin condition comprises a pore distribution, a skin oil and water content, a skin elasticity, and texture, blemishes and acne of the skin.

8. The skin detecting system according to claim 1, wherein the mobile device (2) further comprises a data processing module (24) and a network module (26).

9. The skin detecting system according to claim 1, wherein the network module (26) is provided for sending the information to an outer apparatus (3).

10. The skin detecting system according to claim 8, wherein the outer apparatus (3) comprises a cloud database, a network platform, a detecting instrument, a computer, or other mobile device.

11. The skin detecting system according to claim 8, wherein the mobile device (2) further comprises a USB connector (25), the data processing module (24) transmits the information through the USB connector (25).

## Amended claims

### Amended claims in accordance with Rule 137(2) EPC.

1. A skin detecting system, comprising:
a mobile device (2), comprising a camera (20), an image unit (21), an interpretative software (22), a data processing module (24) and a USB connector (25); and
a close-up photography assembly (1), disposed on the camera (20) of the mobile device (2), the close-up photography assembly (1) comprising a macrolens (10), an electric wire (11), a light source module (12) and an electrical impedance module (13), wherein the macrolens (10) has a pre-determined magnification ratio and autofocuses when operated by the mobile device (10), the electric wire (11) being provided for connecting the close-up photography assembly (1) to the mobile device (2), the light source module (12) being configured to provide a light source required for the close-up photography assembly (1) to photograph and the electrical impedance module (13) being configured for detecting oil and water content in a skin of a human being; wherein the electrical impedance module (13) is configured to transmit its detecting results to the USB connector (25) through the electric wire (11) in the form of signals, the USB connector (25) being configured to further transmit the plurality of detecting result signals to the data processing module (24), and the data processing module (24) being configured to further transmit the received signals to the interpretation software (22), which is configured for executing analysis about the oil and water content of the skin;
wherein when the close-up photography assembly (1) cooperates with the camera (20) of the mobile device (2) to photograph the skin of the human being, the skin of the human being is irradiated by the light source that is provided by the light source module (12) of the close-up photography assembly (1), and then an image corresponding to the skin of the human being is formed by the image unit (21) and shown on a display unit (23) of the mobile device (2), and the interpretative software (22) being configured for adjusting magnification and resolution of the image, and for executing an analysis of a skin condition to acquire an information corresponding to the skin condition for being shown on the display unit (23) of the mobile device (2).

2. The skin detecting system according to claim 1, wherein the light source provided by the light source module (12) is ultraviolet light.

3. The skin detecting system according to claim 1, wherein the light source provided by the light source module (12) is white light.

4. The skin detecting system according to claim 1, wherein the image unit (21) of the mobile device (2) provides ultraviolet light or white light.

5. The skin detecting system according to claim 1, wherein the close-up photography assembly (1) comprises an electric wire (11) configured for electrically connecting to the mobile device (2).

6. The skin detecting system according to claim 1, wherein the analysis of the skin condition comprises a pore distribution, a skin oil and water content, a skin elasticity, and texture, blemishes and acne of the skin.

7. The skin detecting system according to claim 1, wherein the mobile device (2) further comprises a network module (26).

8. The skin detecting system according to claim 1, wherein the network module (26) is configured for sending the information to an outer apparatus (3).

9. The skin detecting system according to claim 7, wherein the outer apparatus (3) comprises a cloud database, a network platform, a detecting instrument, a computer, or other mobile device.

10. The skin detecting system according to claim 7, wherein the data processing module (24) is configured for transmitting the information through the USB connector (25).
